# EUROPÄISCHE PATENTSCHRIFT

(11) **EP 2 820 000 B1**
(45) Veröffentlichungstag und Bekanntmachung des Hinweises auf die Patenterteilung: **20.04.2016**
(21) Anmeldenummer: 13709345.6
(22) Anmeldetag: 28.02.2013
(51) Int. Cl.: C07D 239/84, A61K 31/517, A61P 31/22, C07C 309/29, C07C 309/30

(54) **BESYLAT- UND TOSYLATSALZE EINES DIHYDROCHINAZOLINDERIVATES UND IHRE VERWENDUNG ALS ANTIVIRALE MITTEL**
BESYLATE AND TOSYLATE SALTS OF A DIHYDROQUINAZOLINE DERIVATIVE AND THEIR USE AS ANTIVIRAL AGENTS
SELS DE BÉSYLATE ET DE TOSYLATE D'UN DÉRIVÉ DE DIHYDROQUINAZOLINE ET LEUR UTILISATION EN TANT QU'AGENTS ANTIVIRAUX

(30) Priorität: 29.02.2012 DE 102012101673
(43) Veröffentlichungstag der Anmeldung: 07.01.2015
(73) Patentinhaber: AiCuris Anti-infective Cures GmbH, 42117 Wuppertal (DE)
(72) Erfinder: MÄRTENS, Welljanne, 01127 Dresden (DE); SCHICKANEDER, Christian, 01309 Dresden (DE)
(74) Vertreter: Kilger, Ute
(86) Internationale Anmeldenummer: PCT/EP2013/054109
(87) Internationale Veröffentlichungsnummer: WO 2013/127968

(56) Entgegenhaltungen:
- WO-A1-2004/096778

## Beschreibung

Die vorliegende Erfindung betrifft Salze von {8-Fluor-2-[4-(3-methoxyphenyl)piperazin-1-yl]-3-[2-methoxy-5-(trifluormethyl)phenyl]-3,4-dihydrochinazolin-4-yl}essigsäure und deren Solvate.

Die Erfindung betrifft ferner Verfahren zu ihrer Herstellung, ihre Verwendung zur Behandlung und/oder Prophylaxe von Krankheiten, insbesondere von Virusinfektionen sowie ihre Verwendung zur Herstellung von Arzneimitteln zur Behandlung und/oder Prophylaxe von Virusinfektionen insbesondere zur Behandlung und/oder Prophylaxe von Infektionen mit Cytomegalieviren oder einem anderen Vertreter der Gruppe der Herpes viridae.

{8-Fluor-2-[4-(3-methoxyphenyl)piperazin-1-yl]-3-[2-methoxy-5-(trifluormethyl)phenyl]-3,4-dihydrochinazolin-4-yl}essigsäure ist z.B. aus der WO 2004/096778 bekannt und wurde von der Anmelderin als aussichtsreicher Kandidat für eine antiviral wirksame Substanz insbesondere zur Bekämpfung von Infektionen mit dem humanen Cytomegalievirus (HCMV) entwickelt. Bei der Entwicklung hat es sich allerdings als äußerst kompliziert erwiesen, die Verbindung sei es als Zwitterion, sei es in Salzform in kristalliner Form zu erhalten und bis jetzt erfolgte die Entwicklung unter Verwendung des Zwitterions in amorpher Form. Insbesondere zur Aufreinigung des Wirkstoffs aber auch zum Einsatz in Medikamenten wäre es wünschenswert kristalline Salze von {8-Fluor-2-[4-(3-methoxyphenyl)piperazin-1-yl]-3-[2-methoxy-5-{trifluormethyl}phenyl]-3,4-dihydrochinazolin-4-yl}essigsäure zu erhalten, die auf einfache Weise und in hoher Ausbeute herstellbar sind.

Es ist somit eine Aufgabe der Erfindung, Salze von {8-Fluor-2-[4-(3-methoxyphenyl)piperazin-1-yl]-3-[2-methoxy-5-(trifluormethyl)phenyl]-3,4-dihydro-chinazolin-4-yl}essigsäure zu beschreiben, mit denen kristalline Produkte erhalten werden können. Diese kristallinen Produkte sollten sich insbesondere durch eine hohe Reinheit, sowie eine einfache Herstellbarkeit auszeichnen. Insbesondere wäre es vorteilhaft, wenn Produkte erhalten werden könnten, die weitgehend oder völlig frei von Lösungsmitteln sind.

Überraschenderweise wurde nun herausgefunden, dass {8-Fluor-2-[4-(3-methoxyphenyl)piperazin-1-yl]-3-[2-methoxy-5-(trifluormethyl)phenyl]-3,4-dihydro-chinazolin-4-yl]essigsäure mit Besylat- und Tosylatanionen gut definierte kristalline Salze bildet. Es hat sich ferner gezeigt, dass diese Salze auf einfache Weise in hoher Reinheit hergestellt werden können und frei von Lösungsmitteln auskristallisieren.

Gegenstand der Erfindung sind somit die kristallinen Besylatsalze und kristallinen Tosylatsalze von {8-Fluor-2-[4-(3-methoxyphenyl)piperazin-1-yl]-3-[2-methoxy-5-(trifluormethyl)phenyl]-3,4-dihydrochinazolin-4-yl}essigsäure, sowie deren Solvate.

Besylat- und Tosylatsalze von {8-Fluor-2-[4-(3-methoxyphenyl)piperazin-1-yl]-3-[2-methoxy-5-(trifluormethyl)phenyl]-3,4-dihydrochinazolin-4-yl}essigsäure sind im Rahmen der Erfindung Addukte einer Reaktion der {8-Fluor-2-[4-(3-methoxyphenyl)piperazin-1-yl]-3-[2-methoxy-5-(trifluormethyl)phenyl]-3,4-dihydro-chinazolin-4-yl}essigsäure mit Benzolsulfonsäure bzw. Toluolsulfonsäure. Die {8-Fluor-2-[4-(3-methoxyphenyl)piperazin-1-yl]-3-[2-methoxy-5-(trifluormethyl)phenyl]-3,4-dihydrochinazolin-4-yl}essigsäure und die Besylat- bzw. Tosylatgegenionen können hierbei in jedem Verhältnis vorliegen. Bevorzugt ist das Verhältnis dabei ganzzahlig (z.B. 1:1, 1:2, 1:3, 3:1, 2:1). Die Salze können dabei durch eine direkte Umsetzung der {8-Fluor-2-[4-(3-methoxyphenyl)piperazin-1-yl]-3-[2-methoxy-5-(trifluormethyl)phenyl]-3,4-dihydro-chinazolin-4-yl}essigsäure mit Benzolsulfonsäure oder Toluolsulfonsäure oder durch Herstellung eines anderen Säuresalzes der {8-Fluor-2-[4-(3-methoxyphenyl)piperazin-1-yl]-3-[2-methoxy-5-(trifluormethyl}phenyl]-3,4-dihydrochinazolin-4-yl}essigsäure gefolgt von einem Austausch des Gegenions hergestellt werden.

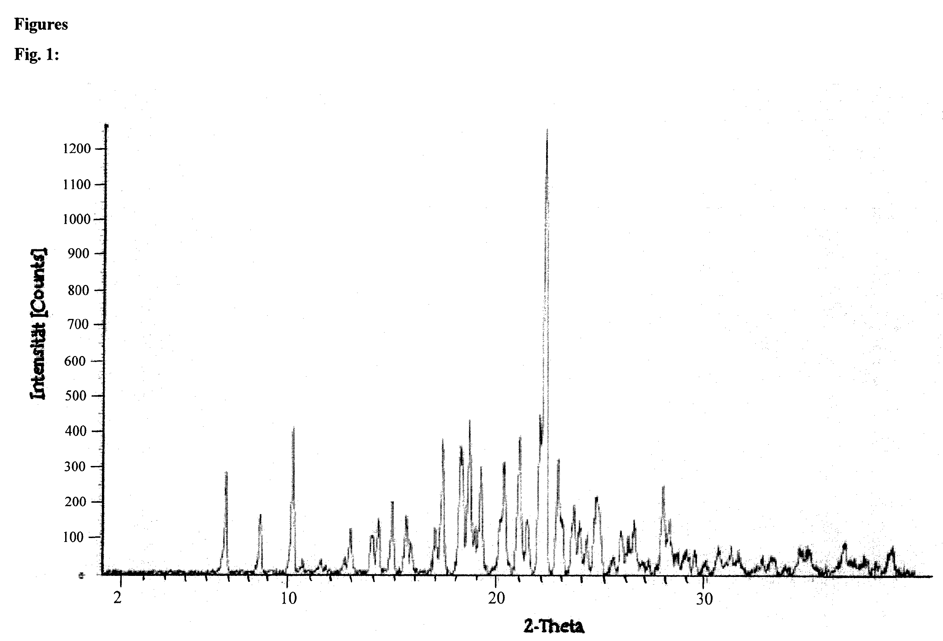

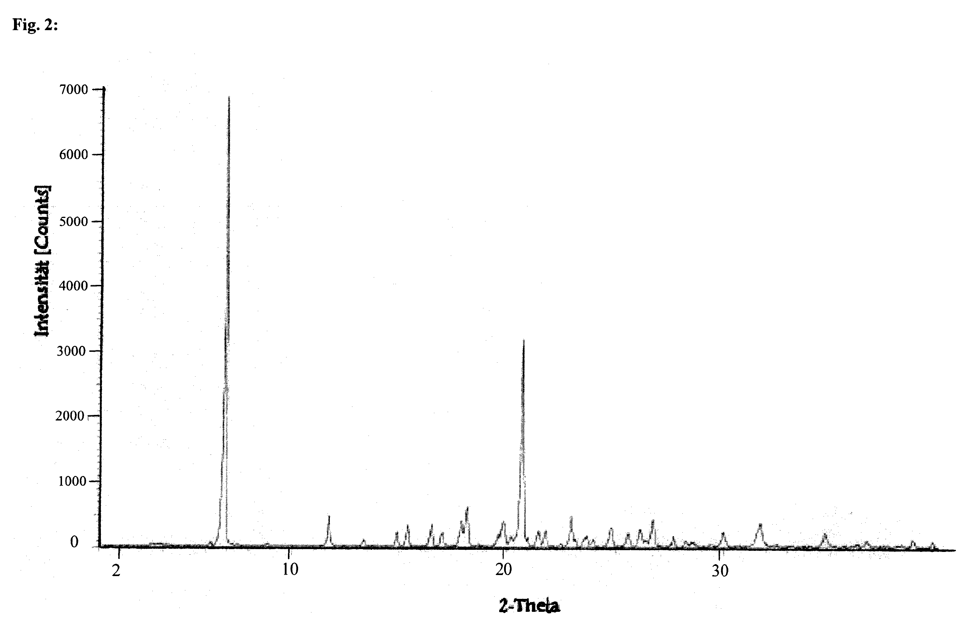

Der Begriff "kristallines Produkt" im Kontext der vorliegenden Erfindung bezeichnet S(+)-{8-Fluor-2-[4-(3-methoxyphenyl)piperazin-1-yl]-3-[2-methoxy-5-(trifluormethyl}-phenyl]-3,4-dihydrochinazolin-4-yl}essigsäure Besylate und S(+}-{8-Fluor-2-[4-(3-methoxyphenyl)piperazin-1-yl]-3-[2-methoxy-5-{trifluormethyl)phenyl]-3,4-dihydro-chinazolin-4-yl}essigsäure Tosylate, welche unter Röntgendiffraktions-Analyse das charakteristische Peak-Muster wie in den entsprechenden Figuren 1 und 2 dargestellt oder ein ähnliches Peak-Muster aufweisen.

Die Begriffe "hohe Reinheit, Reinheit und rein" im Zusammenhang mit den erfindungsgemäßen S(+)-{8-Fluor-2-[4-(3-methoxyphenyl)piperazin-1-yl]-3-[2-methoxy-5-(trifluor-methyl)phenyl]-3,4-dihydrochinazolin-4-yl}essigsäure Besylate und S(+)-{8-Fluor-2-[4-(3-methoxyphenyl)piperazin-1-yl]-3-[2-methoxy-5-{trifluormethyl}phenyl]-3,4-dihydrochinazolin-4-yl}essigsäure Tosylate, bezeichnet dessen Vorhandensein als Stoff in einem Stoffgemisch mit einem < 0,1 %, vorzugsweise < 0,08%, am meisten bevorzugt < 0,05% Gesamtanteil dessen bekannter Verunreinigungen Di-p-Toluoyl-D-Weinsäure, und/oder S-Chinazolylpiperazin, und/oder Chinazolin Ethylester und/oder Chinazolyldipiperazin und/oder dessen unspezifischer Verunreinigungen, wenn mittels HPLC gemäß Methode 4 (siehe Ausführungsbeispiele C.) gemessen wird.

Als Solvate werden im Rahmen der Erfindung solche Formen der Salze von {8-Fluor-2-[4-(3-methoxyphenyl)piperazin-1-yl]-3-[2-methoxy-5-(trifluormethyl)phenyl]-3,4-dihydrochinazolin-4-yl}essigsäure bezeichnet, welche durch Koordination mit Lösungsmittelmolekülen einen Komplex bilden. Hydrate sind eine spezielle Form der Solvate, bei denen die Koordination mit Wasser erfolgt.

Bevorzugt im Rahmen der vorliegenden Erfindung ist das Monobesylatsalz von {8-Fluor-2-[4-(3-methoxyphenyl)piperazin-1-yl]-3-[2-methoxy-5-(trifhiormethyl)phenyl]-3,4-dihydrochinazolin-4-yl}essigsäure. Ferner bevorzugt ist im Rahmen der Erfindung das Monotosylatsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure.

Bevorzugt im Rahmen der Erfindung ist ferner ein Besylatsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das im Pulver-XRD-Diffraktogramm charakteristische Peaks bei etwa 6,9, 10,1 und 22,2 Grad 2Theta zeigt.

Bevorzugt im Rahmen der Erfindung ist ferner ein Tosylatsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das im Pulver-XRD-Diffraktogramm charakteristische Peaks bei etwa 6,9 und 20,7 Grad 2Theta zeigt.

Wie es einem Fachmann ohne Weiteres ersichtlich ist, weist {8-Fluor-2-[4-(3-methoxyphenyl)piperazin-1yl]-3-[2-methoxy-5-(trifluormethyl)phenyl]-3,4-dihydrochinazolin-4-yl}essigsäure am Kohlenstoff in 4-Position des Dihydrochinazolinrings ein Stereozentrum auf. Im Rahmen der vorliegenden Erfindung ist es besonders bevorzugt, wenn dieser Kohlenstoff die S-Konfiguration aufweist.

Die erfindungsgemäßen Salze werden im Allgemeinen durch Umsetzen von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder eines Basensalzes davon mit Benzolsulfonsäure bzw. Toluolsulfonsäure in einem Lösungsmittel hergestellt.

Der Ausdruck "einfache Herstellung" bezieht sich im Kontext der Erfindung auf den Erhalt kristalliner Produkte von S(+)-{8-Fluor-2-[4-(3-methoxyphenyl)piperazin-1-y1]-3-[2-methoxy-5-(trifluormethyl)phenyl]-3,4-dihydrochinazolin-4-yl}essigsäure Besylat und S(+)-{8-Fluor-2-[4-(3-methoxyphenyl)piperazin-1-yl]-3-[2-methoxy-5-(trifluormethyl)-phenyl]-3,4-dihydrochinazolin-4-yl}essigsäure Tosylat durch die oben beschriebene Umsetzung von {8-Fluor-2-[4-(3-methoxyphenyl)piperazin-1-yl]-3-[2-methoxy-5-(trifluor-methyl)phenyl]-3,4-dihydrochinazolin-4-yl}essigsäure oder eines Basensalzes davon mit entsprechend Benzolsulfonsäure bzw. Toluolsulfonsäure in einem Lösungsmittel.

Es ist ferner möglich ein Säuresalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure, bei dem es sich nicht um eine Besylat- oder Tosylatsalz handelt, mit einer Quelle für Besylat- oder Tosylatanionen in einem Lösungsmittel umzusetzen.

Insbesondere wird bei den oben erwähnten Umsetzungen als Lösungsmittel eine Mischung aus Wasser und zumindest einem (C₃-C₆)-Alkanon eingesetzt.

Gegenstand der Erfindung ist somit auch ein Verfahren zur Herstellung eines Besylat- oder Tosylatsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure mit den folgenden Schritten:
a.) Lösen von {8-Fluor-2-[4-(3-methoxyphenyl)piperazin-1-yl]-3-[2-methoxy-5-(trifluormethyl)phenyl]-3,4-dihydrochinazolin-4-yl}-essigsäure oder eines Solvats davon in einer Mischung aus Wasser und zumindest einem (C₃-C₆)-Alkanon, ggf. unter Erwärmen.
b.) Zugabe von Benzolsulfonsäure oder Toluolsulfonsäure zu der in Schritt a.) erhaltenen Lösung,
c.) Abkühlen der in Schritt b.) erhaltenen Lösung um die Kristallisation des Salzes bzw. eines Solvat des Salzes auszulösen,
d.) Abtrennen des in Schritt c.) auskristallisierten Salzes oder Solvats davon, und
e.) Trocknen des in Schritt d.) erhaltenen Salzes oder Solvats.

Die so erhaltenen erfindungsgemäßen Salze können ggf. weiterbearbeitet, z.B. umkristallisiert oder mikronisiert werden, um deren physikalische Eigenschaften weiter auf den Verwendungszweck anzupassen.

Bevorzugt werden die erfindungsgemäßen Salze auch zur Aufreinigung von {8-Fluor-2-[4-{3-methoxyphenyl)piperazin-1-yl]-3-[2-methoxy-5-(trifluormethyl}phenyl]-3,4-dihydro-chinazolin-4-yl}essigsäure verwendet. Hierzu wird aufzureinigende {8-Fluor-2-[4-(3-methoxyphenyl)piperazin-1-yl]-3-[2-methoxy-5-(trifluormethyl)phenyl]-3,4-dihydro-chinazolin-4-yl}essigsäure in einem Lösungsmittel mit Benzolsulfonsäure oder Toluolsulfonsäure umgesetzt, das entstehende kristalline Salz isoliert und die zwitterionische Form der {8-Fluor-2-[4-(3-methoxyphenyl)piperazin-1-yl]-3-[2-methoxy-5-(trifluormethyl)phenyl]-3,4-dihydrochinazolin-4-yl}essigsäure durch Behandeln des Salzes mit einer Pufferlösung bei pH 5 bis 7 wieder freigesetzt.

Gegenstand der Erfindung ist somit auch ein Verfahren zur Aufreinigung von {8-Fluor-2-[4-(3-methoxyphenyl)piperazin-1yl]-3-[2-methoxy-5-(trifluormethyl)phenyl]-3,4-dihydrochinazolin-4-yl}essigsäure mit den folgenden Schritten:
1.) Umsetzen von {8-Fluor-2-[4-(3-methoxyphenyl)piperazin-1-yl]-3-[2-methoxy-5-(trifluormethyl)phenyl]-3,4-dihydrochinazolin-4-yl}essigsäure mit Benzolsulfonsäure bzw. Toluolsulfonsäure in einem Lösungsmittel um ein kristallines Salz zu erhalten,
2.) Isolieren des in Schritt 1.) erhaltenen Salzes,
3.) Behandeln des in Schritt 2.) isolierten Salzes mit einer Pufferlösung bei pH 5 bis 7 zum Freisetzen der zwitterionischen Form von {8-Fluor-2-[4-(3-methoxyphenyl)piperazin-1-yl]-3-[2-methoxy-5-(trifluormethyl)phenyl]-3,4-dihydrochinazolin-4-yl}essigsäure, und
4.) Isolieren der in Schritt 3.) erhaltenen zwitterionischen Form von {8-Fluor-2-[4-(3-methoxyphenyl)piperazin-1-yl]-3-[2-methoxy-5-(trifluormethyl)phenyl]-3,4-dihydrochinazolin-4-yl}essigsäure.

Die zur Herstellung der erfindungsgemäßen Salze verwendete {8-Fluor-2-[4-(3-methoxyphenyl)piperazin-1-yl]-3-[2-methoxy-5-(trifluormethyl)phenyl]-3,4-dihydrochinazolin-4-yl}essigsäure ist bekannt und kann zum Beispiel nach dem in der WO 2006/133822 beschriebenen Verfahren hergestellt werden.

Insbesondere erfolgt die Herstellung durch die Verseifung des Esters einer Verbindung der Formel (II) 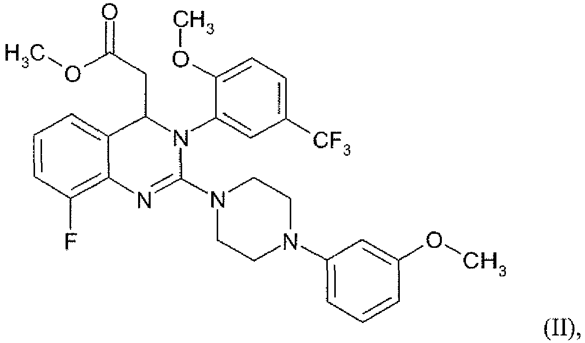 mit einer Base.

Die Verbindung der Formel (II) kann hergestellt werden durch Umsetzung einer Verbindung der Formel (III) 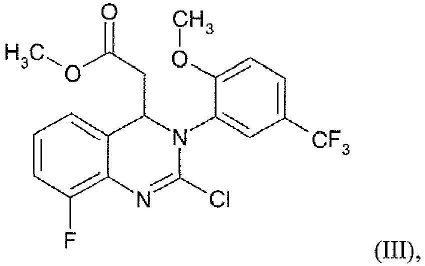 in Gegenwart einer Base mit einer Verbindung der Formel (IV)

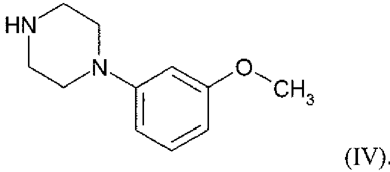

Die Verbindung der Formel (III) kann hergestellt werden durch Umsetzung einer Verbindung der Formel (V) 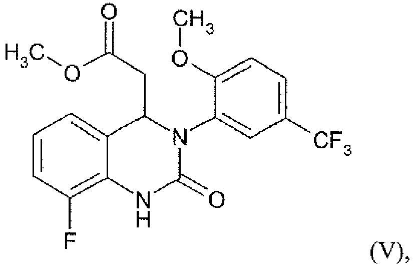 mit Phosphoroxychlorid, Phosphortrichlorid oder Phosphorpentachlorid in Gegenwart einer Base.

Die Verbindung der Formel (V) kann hergestellt werden durch Umsetzung einer Verbindung der Formel (VI) 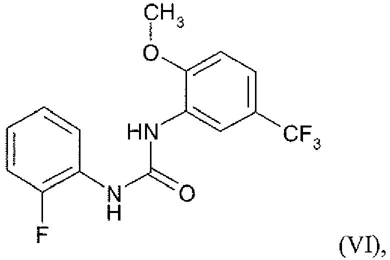 in der ersten Stufe mit Acrylsäuremethylester in Gegenwart eines Palladiumkatalysators und Oleum und in der zweiten Stufe mit einer Base.

Verbindungen der Formeln (IV) und (VI) sind dem Fachmann an sich bekannt oder lassen sich nach üblichen, literaturbekannten Verfahren herstellen.

Die Verseifung des Esters einer Verbindung der Formel (II) zu {8-Fluor-2-[4-(3-methoxyphenyl)piperazin-1-yl]-3-[2-methoxy-5-(trifluormethyl)phenyl]-3,4-dihydrochinazolin-4-yl}essigsäure erfolgt durch Umsetzung einer Verbindung der Formel (II) mit einer Base in einem inerten Lösungsmittel, in einem Temperaturbereich von 18°C bis zum Rückfluss des Lösungsmittels, bevorzugt bei 18 bis 50°C, besonders bevorzugt bei 20 bis 30°C, bei Normaldruck, innerhalb von beispielsweise 0,5 bis 10 Stunden, bevorzugt innerhalb von 1 bis 5 Stunden.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Natrium- oder Kaliummethanolat oder Natrium- oder Kaliumethanolat, wobei die Base ggf. in wässriger Lösung vorliegt.

Inerte Lösungsmittel sind beispielsweise Ether wie 1,2-Dimethoxyethan, Methyl-*tert-*butylether (MTBE), Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Wasser, oder Gemische von Lösungsmitteln.

Bevorzugt ist Natriumhydroxid in Wasser und MTBE.

Die Synthese einer Verbindung der Formel (II) aus einer Verbindung der Formel (III) und einer Verbindung der Formel (IV) in Gegenwart einer Base erfolgt in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 2 bis 48 Stunden, bevorzugt innerhalb von 4 bis 12 Stunden.

Basen sind beispielsweise Aminbasen wie 1,8-Diazabicyclo[5.4.0]undec-7-en (DBU), 1-(3-Methoxyphenyl)piperazin oder Triethylamin, oder andere Basen wie Kalium-tert.-butylat.

Inerte Lösungsmittel sind beispielsweise Chlorbenzol oder Ether wie 1,2-Dimethoxyethan, Dioxan, Glykoldimethylether oder Diethylenglykoldimethylether.

Bevorzugt ist DBU in Dioxan.

Die Umsetzung einer Verbindung der Formel (V) zu einer Verbindung der Formel (III) erfolgt durch Reaktion einer Verbindung der Formel (V) mit Phosphoroxychlorid, Phosphortrichlorid oder Phosphorpentachlorid, bevorzugt ist Phosphoroxychlorid, in Gegenwart einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 1 bis 48 Stunden, bevorzugt innerhalb von 2 bis 12 Stunden.

Basen sind beispielsweise Amine wie 1,8-Diazabicyclo[5.4.0]undec-7-en (DBU), Pyridin oder Triethylamin, oder andere Basen wie Kalium-tert.-butylat.

Inerte Lösungsmittel sind beispielsweise Kohlenwasserstoffe wie Benzol, Xylol, Toluol oder Chlorbenzol.

Bevorzugt ist DBU in Chlorbenzol.

Die Umsetzung einer Verbindung der Formel (VI) zu einer Verbindung der Formel (V) erfolgt in der ersten Stufe durch Reaktion einer Verbindung der Formel (VI) mit Acrylsäuremethylester in Gegenwart eines Palladiumkatalysators und Oleum in einem Lösungsmittel in einem Temperaturbereich von 0°C bis 40°C, bevorzugt bei Raumtemperatur und in der zweiten Stufe durch Reaktion mit einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 1 bis 48 Stunden, bevorzugt innerhalb von 2 bis 12 Stunden.

Palladiumkatalysatoren in der ersten Stufe sind beispielsweise Palladium(II)acetat, Bis(triphenylphosphin)palladium(II)chlorid, Tetrakis(triphenylphosphin)palladium(0), Bis(tris(o-tolyl)phosphino)palladium-(II)-chlorid oder ein aus Bis-(acetonitril)dichlorpalladium oder Palladium(II)acetat und einem Liganden, beispielsweise Tris(o-tolyl)phosphin, Triphenylphosphin oder Diphenylphosphinoferrocen, hergestellter Palladiumkatalysator.

Lösungsmittel in der ersten Stufe sind beispielsweise organische Säuren wie Essigsäure oder Propionsäure.

Bevorzugt ist Palladium(II)acetat in Essigsäure.

Basen in der zweiten Stufe sind beispielsweise DBU, Triethylamin oder Diisopropylethylamin.

Inerte Lösungsmittel in der zweiten Stufe sind beispielsweise Ether wie 1,2-Dimethoxyethan, Dioxan, Glykoldimethylether oder Diethylenglykoldimethylether, Kohlenwasserstoffe wie Benzol, Xylol oder Toluol, oder andere Lösungsmittel wie Isobutyronitril, Acetonitril, Aceton, Nitrobenzol, Dimethylformamid, Dimethylacetamid, Dimethylsulfoxid oder N-Methylpyrrolidon.

Bevorzugt ist DBU in Aceton.

Die Herstellung der zur Herstellung der erfindungsgemäßen Salze verwendeten {8-Fluor-2-[4-(3-methoxyphenyl)piperazin-1-yl]-3-[2-methoxy-5-(trifluormethyl)phenyl]-3,4-dihydrochinazolin-4-yl}essigsäure ist in dem nachstehenden Syntheseschema 1 exemplarisch näher erläutert. Das Syntheseschema ist hierbei rein beispielhaft und in keiner Weise einschränkend zu verstehen.

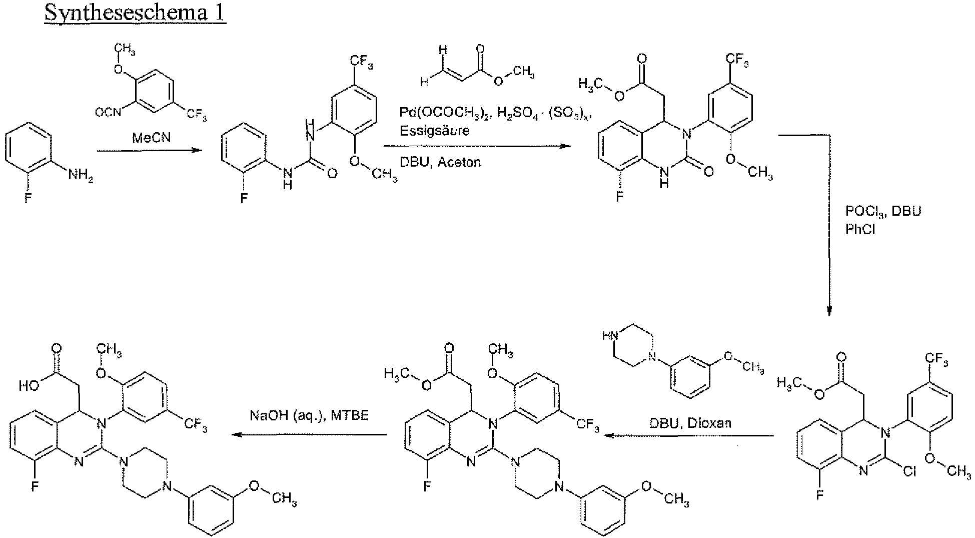

Wie bereits zuvor erwähnt wird die {8-Fluor-2-[4-(3-methoxyphenyl)piperazin-1-yl]-3-[2-methoxy-5-(trifluormethyl)phenyl]-3,4-dihydrochinazolin-4-yl}essigsäure vorzugsweise in Form des S-Enantiomers eingesetzt. Dieses S-Enantiomer kann dabei z.B. wie in dem nachstehenden Syntheseschema 2 dargestellt hergestellt werden.

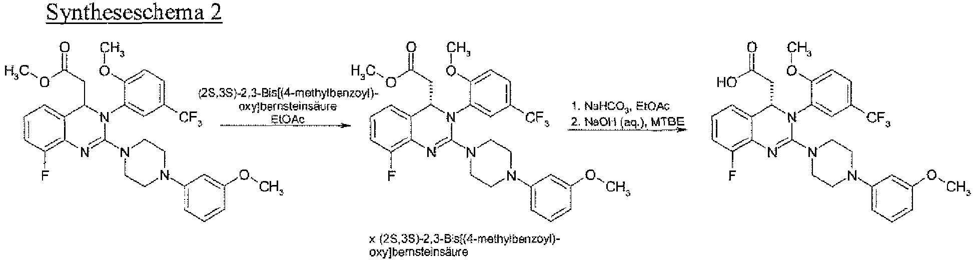

Die erfindungsgemäßen Salze zeigen eine antivirale Wirkung gegenüber Vertretern der Gruppe der Herpes viridae (Herpesviren), vor allen Dingen gegenüber Cytomegalieviren (CMV) insbesondere gegenüber dem humanen Cytomegalievirus (HCMV). Sie sind somit geeignet zur Behandlung und Prophylaxe von Krankheiten, vor allem von Infektionen mit Viren, insbesondere den hierin genannten Viren und den dadurch hervorgerufenen Infektionskrankheiten. Unter einer Virusinfektion wird hierin sowohl eine Infektion mit einem Virus als auch eine durch eine Infektion mit einem Virus hervorgerufene Krankheit verstanden.

Die erfindungsgemäßen Salze können aufgrund ihrer Eigenschaften zur Herstellung von Arzneimitteln verwendet werden, die zur Prophylaxe und/oder Behandlung von Krankheiten, insbesondere Virusinfektionen geeignet sind.

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.
6) Behandlung von HCMV-positiven Krebspatienten mit dem Ziel, HCMV-vermittelte Tumorprogression zu verringern (vgl. J. Cinatl , et al., FEMS Microbiology Reviews 2004, 28, 59-77).

Bevorzugt werden die erfindungsgemäßen Salze zur Herstellung von Arzneimitteln verwendet, die zur Prophylaxe und/oder Behandlung von Infektionen mit einem Vertreter der Gruppe der Herpes viridae, besonders einem Cytomegalievirus, insbesondere dem humanen Cytomegalievirus, geeignet sind.

Die erfindungsgemäßen Salze können aufgrund ihrer pharmakologischen Eigenschaften allein und bei Bedarf auch in Kombination mit anderen Wirkstoffen, insbesondere antiviralen Wirkstoffen wie beispielsweise Valganciclovir, Ganciclovir, Valacyclovir, Acyclovir, Foscarnet, Cidofovir und verwandten Derivaten zur Behandlung und/oder Prävention von Virusinfektionen, insbesondere von HCMV-Infektionen, eingesetzt werden.

Weiterer Gegenstand der vorliegenden Erfindung ist der Einsatz der erfindungsgemäßen Salze in einem Verfahren zur Behandlung und/oder Prophylaxe von Erkrankungen, vorzugsweise von Virusinfektionen, insbesondere von Infektionen mit dem humanen Cytomegalievirus (HCMV) oder einem anderen Vertreter der Gruppe der Herpes viridae.

Weiterer Gegenstand der vorliegenden Erfindung ist die Verwendung der erfindungsgemäßen Salze zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Salze zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antiviral wirksamen Menge der erfindungsgemäßen Salze.

Die erfindungsgemäßen Salze können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Salze in geeigneten Applikationsformen verabreicht werden.

Für die orale Applikation eignen sich nach dem Stand der Technik funktionierende schnell und/oder modifiziert die erfindungsgemäßen Salze abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Salze können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglykole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens ein erfindungsgemäßes Salz, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei oraler Applikation Mengen bezogen auf den reinen Wirkstoff von etwa 0,01 bis 25 mg/kg, vorzugsweise etwa 0,1 bis 10 mg/kg Körpergewicht zur Erzielung wirksamer Ergebnisse zu verabreichen.

Trotzdem kann es ggf. erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun nachstehend anhand von Beispielen sowie in Bezug auf die beiliegenden Zeichnungen näher erläutert. Es zeigen:
- Fig.: 1 ein Pulver XRD-Diffraktogramm eines Besylatsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das gemäß Beispiel 1 hergestellt wurde; und
- Fig. 2: ein Pulver XRD-Diffraktogramm eines Tosylatsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das gemäß Beispiel 2 hergestellt wurde.
- Fig.: 3 HPLC-Analyse von S(+)-{8-Fluor-2-[4-(3-methoxyphenyl}piperazin-1-yl]-3-[2-methoxy-5-(trifluormethyl)phenyl]-3,4-dihydrochinazolin-4-yl}essigsäure Besylat mittels relativer Flächenprozent-Methode mit Einbeziehung der entsprechenden Response-Faktoren (RF); Peak-Name, Retentionszeit, relative Flächenprozent (mit RF) % in Tabellenform.

- Fig. 4: Chromatogramm HPLC-Reinheit S(+)-{8-Fluor-2-[4-(3-methoxyphenyl)-piperazin-1-yl]-3-[2-methoxy-5-(trifluormethyl)phenyl]-3,4-dihydrochinazolin-4-yl}essigsäure Besylat.
- Fig. 5: HPLC-Analyse von S(+)-{8-Fluor-2-[4-(3-methoxyphenyl)piperazin-1-yl]-3-[2-methoxy-5-{trifluormethyl)phenyl}-3,4-dihydrochinazolin-4-yl}essigsäure Tosylat mittels relativer Flächenprozent-Methode mit Einbeziehung der entsprechenden Response-Faktoren (RF); Peak-Name, Retentionszeit, relative Flächenprozent (mit RF) % in Tabellenform.
- Fig. 6: Chromatogramm HPLC-Reinheit S(+)-{8-Fluor-2-[4-(3-methoxyphenyl)-piperazin-1-yl]-3-[2-methoxy-5-(trifluormethyl)phenyl]-3,4-dihydrochinazolin-4-yl}essigsäure Tosylat.

Die Prozentangaben in den folgenden Tests und Beispielen sind, sofern nicht anders angegeben, Gewichtsprozent, Teile sind Gewichtsteile. Lösungsmittelverhältnisse, Verdünnungsverhältnisse und Konzentrationsangaben von Flüssiglösungen beziehen sich jeweils auf das Volumen.

### Abkürzungsverzeichnis:

- ACN: Acetonitril
- API: active pharmaceutical ingredient
- API-ES-pos.: Atmospheric pressure ionization, electrospray, positive (bei MS)
- API-ES-neg.: Atmospheric pressure ionization, electrospray, negative (bei MS)
- ca.: circa
- CI, NH₃: chemische Ionisierung (mit Ammoniak)
- DBU: 1,8-Diazabicyclo[5.4.0]undec-7-en
- DMAP: 4-(Dimethylamino)pyridin
- DMSO: Dimethylsulfoxid
- ESTD: externe Standardisierung
- h: Stunde(n)
- HPLC: Hochdruckflüssigchromatographie (high pressure liquid chromatography)
- konz.: konzentriert(e)
- min.: Minuten
- MS: Massenspektroskopie
- MTBE: Methyl-*tert*-butylether
- NMR: Kernresonanzspektroskopie (nuclear magnetic resonance spectroscopy)
- R_{T}: Retentionszeit (bei HPLC)
- VTS: Vakuumtrockenschrank

### Allgemeine Methoden HPLC:

**Methode 1** (HPLC): Instrument: HP 1050 mit variabler Wellenlängen-Detektion; Säule: Phenomenex-Prodigy ODS (3) 100A, 150 mm x 3 mm, 3 µm; Eluent A: (1,0 g KH₂PO₄ + 1,0 mL H₃PO₄) / 1 Wasser, Eluent B: Acetonitril; Gradient: 0 min 10% B, 25 min 80% B, 35 min 80% B; Fluss: 0,5 ml/min; Temp.: 45°C; UV-Detektion: 210 nm.
**Methode 2** (HPLC): Instrument: HP 1050 mit variabler Wellenlängen-Detektion; Säule: Chiral AD-H, 250 mm x 4,6 mm, 5 µm; Eluent A: *n*-Heptan + 0,2 % Diethylamin, Eluent B: Isopropanol + 0,2 % Diethylamin; Gradient: 0 min 12,5 % B, 30 min 12,5 % B; Fluss: 1 ml/min; Temp.: 25°C; UV-Detektion: 250 nm.
**Methode 3** (HPLC): Instrument: HP 1050 mit variabler Wellenlängen-Detektion; Säule: Chiral AD-H, 250 mm x 4,6 mm, 5 µm; Eluent A: n-Heptan + 0,2 % Diethylamin, Eluent B: Isopropanol + 0,2 % Diethylamin; Gradient: 0 min 25 % B, 15 min 25 % B; Fluss: 1 ml/min; Temp.: 30°C; UV-Detektion: 250 nm.

### Beispiele

### A.) Herstellung von {8-Fluor-2-[4-(3-methoxyphenyl)piperazin-1-yl]-3-[2-methoxy-5-(trifluormethyl)phenyl]-3,4-dihydrochinazolin-4-yl}essigsäure

### Beispiel 1A

### N-(2-Fluorphenyl)-N'-[2-methoxy-5-(trifluormethyl)phenyl]harnstoff

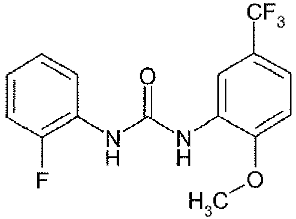

2-Methoxy-5-trifluormethylphenylisocyanat (78 kg) wird bei ca. 35°C ausgeschmolzen und in Acetonitril (insgesamt ca. 2701) gelöst, dann wird 2-Fluoranilin (39,9 kg) zugegeben und mit Acetonitril (ca. 251) nachgespült. Die resultierende klare Lösung wird 4 h unter Rückfluss gerührt und dann auf ca. 75°C abgekühlt. Bei dieser Temperatur wird die Lösung mit Impfkristallen des gewünschten Endprodukts (200 g) angeimpft, für weitere 15 min. gerührt und dann über 3 h auf 0°C abgekühlt. Das erhaltene kristalline Produkt wird durch Zentrifugieren isoliert mit kaltem Acetonitril (zweimal ca. 13 1) gewaschen und bei 45°C im VTS mit Schleppstickstoff getrocknet (ca. 3,5 h). So werden insgesamt 101,5 kg N-*(2-Fluorphenyl)*-*N'*-*[2-methoxy-5-(trifluormethyl)phenyl]harnstoff* als Feststoff erhalten, entsprechend 85,9 % der Theorie.
¹H NMR (300 MHz, d₆-DMSO): δ = 8,93 (s, 1H), 8,84 (s, 1H), 8,52 (d, ³*J* = 2,3, 2H), 7,55 (d, ² *J* = 7,7, 1H), 7,38-7,26 (m, 3H), 7,22 (d, ²*J* = 8,5, 1H), 4,00 (s, 3H) ppm;
MS (API-ES-pos.): m/z = 409 [(M+H)⁺, 100 %];
HPLC (Methode 1): R_{T} = 22,4 und 30,6 min.

### Beispiel 2A

### Methyl-(2Z)-3-[3-fluor-2-({[2-methoxy-5-(trifluormethyl)phenyl]carbamoyl}amino)-phenyl]acrylat

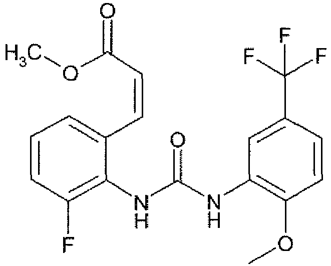

In einem ersten Reaktor werden *N*-(2-Fluorphenyl)-*N'*-[2-methoxy-5-(trifluormethyl)phenyl]hamstoff(51 kg) unter einer Stickstoff-Atmosphäre in Essigsäure (ca. 4301) gelöst. Zu der entstandenen Lösung wird Methylacrylat (20,1 kg) zugegeben und die entstandene Suspension wird bis zur weiteren Verwendung gerührt. In einem zweiten Reaktor wird Essigsäure (950 1) vorgelegt, Oleum (57 kg) vorsichtig zugegeben und Palladium(II)acetat (7 kg) in der erhaltenen Mischung gelöst. Die in dem ersten Reaktor gebildete Suspension wird nun über ca. 2 h zu der in dem zweiten Reaktor enthaltenen Mischung zugegeben, wobei das Reaktionsgemisch mit einem Gemisch aus 96% Stickstoff und 4% Sauerstoff überströmt wird und die erhaltene Reaktionsmischung wird für ca. 18 h bei Raumtemperatur gerührt. Anschließend wird ein Teil der Essigsäure (ca. 9001) abdestilliert, zu der verbleibenden Reaktionsmischung wird über ca. 1 h Wasser (ca. 5001) zugegeben und die erhaltene Suspension wird für 1 h gerührt. Der erhaltene Feststoff wird abfiltriert, einmal mit einem Gemisch aus Essigsäure und Wasser (1:1) und zweimal mit Wasser gewaschen und anschließend bei ca. 30 mbar und 50°C getrocknet. So werden insgesamt 44,8 kg *Methyl-(2Z)-3-[3-fluor-2-({[2-methoxy-5-(trifluormethyl)phenyl]carbamoyl}-amino)phenyl]acrylat* als Feststoff erhalten, entsprechend 65,0 % der Theorie.
¹H NMR (300 MHz, d₆-DMSO): δ = 9,16 (s, 1H), 8,84 (s, 1H), 8,45 (d, 1,7Hz, 1H), 7,73 (m, 2H), 7,33 (m, 3H), 7,22 (d, 8,6Hz, 1H), 6,70 (d, 16Hz, 1H), 3,99 (s, 3H), 3,71 (s, 3H) ppm;
MS (API-ES-pos.): m/z = 429,9 [(M+NH₄)⁺]; 412,9 [(M+H)⁺]
HPLC : R_{T} = 46,4 min.

Instrument: HP 1100 mit variabler Wellenlängen-Detektion; Säule: Phenomenex-Prodigy ODS (3) 100A, 150 mm x 3 mm, 3 µm; Eluent A: (1,36 g KH₂PO₄ + 0,7 ml H₃PO₄) / 1 Wasser, Eluent B: Acetonitril; Gradient: 0 min 20% B, 40 min 45% B, 50 min 80% B, 65 min 80% B; Fluss: 0,5 ml/min; Temp.: 55°C; UV-Detektion: 210 nm.

### Beispiel 3A

### {8-Fluor-3-[2-methoxy-5-(trifluormethym)phenyl]-2-oxo-1,2,3,4-tetrahydrochinazolin-4-yl} essigsäuremethylester

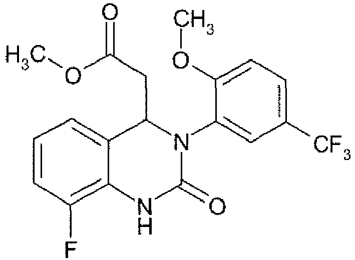

Die Verbindung von Beispiel 2A (75 kg) wird in Aceton (16001) suspendiert und DBU (5,7 kg) wird zugegeben. Die resultierende Suspension wird zum Rückfluss erwärmt und 4 h unter Rückfluss gerührt. Die erhaltene Lösung wird auf eine Manteltemperatur von 55°C abgekühlt und über Kieselgur filtriert. Ein Teil des Lösungsmittels wird durch Destillation aus dem Reaktionsgemisch entfernt (ca. 1125 1) und der verbleibende Rückstand wird für 2 h auf 0°C abgekühlt. Der entstandene Feststoff wird durch Zentrifugieren abgetrennt und zweimal mit kaltem Aceton (ca. 151) gewaschen und über Nacht bei 45°C unter reduziertem Druck mit Schleppstickstoff bis zur Massenkonstanz getrocknet. So werden insgesamt 58,3 kg *{8-Fluor-3-[2-methoxy-5-(trifluormethyl)phenyl]-2-oxo-1,2,3,4-tetrahydrochinazolin-4-yl}essigsäure-methylester* als Feststoff erhalten, entsprechend 84,1 % der Theorie.
HPLC (Methode 1): R_{T} = 19,4 min.

### Beispiel 4A

### (2S,3S)-2,3-Bis[(4-methylbenzoyl)oxy]bernsteinsäure-{(4S)-8-Fluor-2-[4-(3-methoxyphenyl)-piperazin-1-yl]-3-[2-methoxy-5-(trifluormethyl)phenyl]-3,4-dihydrochinazolin-4-yl}-essigsäuremethylester (1:1-Salz) Chlorierung/Aminierung/Kristallisation

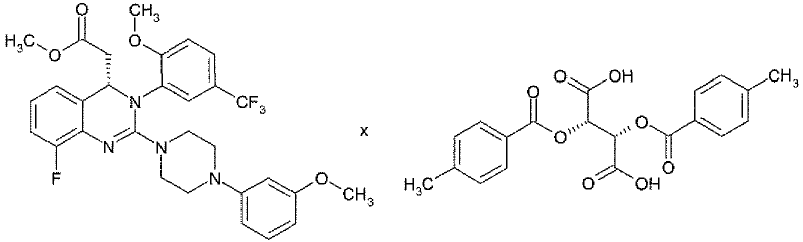

Eine Lösung von {8-Fluor-3-[2-methoxy-5-(trifluormethyl)phenyl]-2-oxo-1,2,3,4-tetrahydrochinazolin-4-yl}essigsäuremethylester (Beispiel 3A, 129,2 kg) in Chlorbenzol (800 1) wird auf Rückfluss erhitzt und azeotrop getrocknet. Phosphoroxychlorid (144 kg) wird zugegeben, und die Reaktionsmischung wird 3 h unter Rückfluss gerührt. Anschließend wird DBU (95 kg) und Chlorbenzol (45 1) zugegeben und für weitere 9 h unter Rückfluss gerührt. Die Reaktionsmischung wird auf Raumtemperatur abgekühlt, durch Zugabe in Wasser hydrolysiert, mit Chlorbenzol (80 1) verdünnt und mit wässriger Ammoniaklösung (25%) neutralisiert. Die Phasen werden getrennt und die organische Phase mit Wasser gewaschen und das Lösungsmittel abdestilliert. Der verbleibende Rückstand wird in Dioxan (170 1) gelöst. 3-Methoxyphenylpiperazin (66 kg), DBU (52 kg) und weitere 90 1 Dioxan werden zugegeben und die Reaktionsmischung wird 4 h unter Rückfluss erwärmt. Die Reaktionsmischung wird auf Raumtemperatur abgekühlt, mit Essigester (1300 1) versetzt, 1x mit Wasser, 3 x mit 0.2 N HCl, und 1x mit wässriger NaCl-Lösung gewaschen und das Lösungsmittel wird abdestilliert. Der erhaltene Rückstand wird in Essigester (800 1) gelöst und in eine Lösung aus (2*S*,3*S*)-2,3-Bis[(4-methylbenzoyl)oxy]bernsteinsäure (121 kg) in Essigester (600 l) gegeben. Das resultierende Gemisch wird ca. 60 min. bei Raumtemperatur gerührt, dann wird mit (2*S*,3*S*)-2,3-Bis[(4-methylbenzoyl)oxy]-bernsteinsäure-{(4*S*)-8-Fluor-2-[4-(3-methoxyphenyl)piperazin-1-yl]-3-[2-methoxy-5-(trifluormethyl)-phenyl]-3,4-dihydrochinazolin-4-yl}essigsäuremethylester angeimpft und 3 Tage bei Raumtemperatur gerührt. Anschließend wird auf 0 - 5°C gekühlt und weitere 3 h gerührt. Die Suspension wird abgesaugt und portionsweise mit Ethylacetat nachgewaschen. So werden insgesamt etwa 141 kg (berechnet als trocken) des Salzes als Feststoff erhalten, entsprechend etwa 46,2 % der Theorie über drei Stufen (Chlorierung, Aminierung und Kristallisation) bezogen auf das Racemat.
¹H NMR (300 MHz, d₆-DMSO): δ = 7,90 (d, ²*J* = 7,8, 4H), 7,56 (d, ²*J* = 8,3, 1H), 7,40 (d, ²*J* = 7,8, 4H), 7,28-7,05 (m, 4H), 6,91-6,86 (m, 2H), 6,45 (d, ²*J* = 8,3, 1H), 6,39-6,36 (m, 2H), 5,82 (s, 2H), 4,94 (m, 1H), 4,03 (q, ²*J* = 7,1, 2H), 3,83 (brs, 3H), 3,69 (s, 3H), 3,64 (s, 3H), 3,47-3,36 (m, 8H und Wasser, 2H), 2,98-2,81 (m, 5H), 2,58-2,52 (m, 1H), 2,41 (s, 6H), 1,99 (s, 3H), 1,18 (t, ²*J* = 7,2, 3H) ppm;
HPLC (Methode 1): R_{T} = 16,6 und 18,5 min.

### Beispiel 5A

### (2S,3S)-2,3-Bis[(4-methylbenzoyl)oxy]bernsteinsäure-{(4S)-8-Fluor-2-[4-(3-methoxyphenyl)piperazin-1-yl]-3-[2-methoxy-5-(trifluormethyl)phenyl]-3,4-dihydro-chinazolin-4-yl}essigsäuremethylester (1:1-Salz) / Umkristallisation

(2S,3S)-2,3-Bis[(4-methylbenzoyl)oxy]bernsteinsäure - (S) {(4*S*)-8-Fluor-2-[4-(3-methoxyphenyl)piperazin-1-yl]-3-[2-methoxy-5-(trifluormethyl)phenyl]-3,4-dihydrochin-azolin-4-yl}essigsäuremethylester (1:1-Salz) (141 kg, berechnet als trocken) wird in Ethylacetat (1400 1) suspendiert und durch Erhitzen auf Rückfluss (77°C) gelöst. Die Lösung wird filtriert und langsam auf Raumtemperatur gekühlt. Dabei erfolgt eine spontane Kristallisation. Die Suspension wird 16 h bei RT gerührt, anschließend auf 0-5°C gekühlt und weitere 3 h gerührt. Die Suspension wird abgesaugt und mit kaltem Ethylacetat nachgewaschen. Die Kristalle werden 16 h im Vakuum bei etwa 40 °C getrocknet. So werden insgesamt 131,2 kg des Salzes als Feststoff erhalten, entsprechend 93,0 % der Theorie.
HPLC (Methode 1): R_{T} = 16,9 und 18,8 min.;
HPLC (Methode 3): 99,9 % e.e.

### Beispiel 6A

### (S)-{8-Fluor-2-[4-(3-methoxyphenyl)piperazin-1-yl]-3-(2-methoxy-5-trifluormethyl-phenyl)-3,4-dihydrochinazolin-4-yl}essigsäure

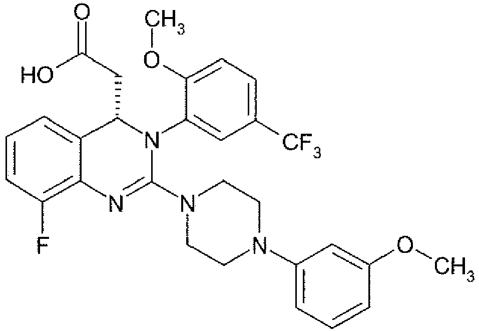

Eine Mischung aus (2*S*,3*S*)-2,3-Bis[(4-methy]benzoyl)oxy]bernsteinsäure-{(4*S*)-8-Fluor-2-[4-(3-methoxyphenyl)piperazin-1-yl]-3-[2-methoxy-5-(trifluormethyl)phenyl]-3,4-dihydro-chinazolin-4-yl}essigsäuremethylester (1:1-Salz) (30,8 kg), Natriumhydrogencarbonat (16.4 kg) und Wasser (3151) wird mit MTBE (1601) verrührt. Die Phasen werden getrennt und die organische Phase mit 35 1 einer etwa siebenprozentigen wässrigen Natriumhydrogencarbonatlösung behandelt. Die Phasen werden getrennt und die organische Phase wird mit 125 1 einer etwa vierprozentigen wässrigen Natriumhydroxidlösung versetzt. Die Reaktionsmischung wird zum Rückfluss erhitzt, das Lösungsmittel schonend bis zum Versiegen destilliert und der Reaktorinhalt anschließend weitere 5 h bei 55 - 60 °C gerührt. Die Reaktionsmischung wird dann bei etwa 22 °C mit MTBE (1601) und Wasser (65 l) versetzt und verrührt. Die Phasen werden getrennt und die organische Phase mit einer etwa sechsprozentigen wässrigen Natriumchloridlösung (30 l) extrahiert. Die vereinigten wässrigen Phasen werden mit Wasser (25 l) und MTBE (160 l) verrührt und der pH-Wert mit etwa 1N Salzsäure auf etwa 6,5 gestellt. Die organische Phase wird abgetrennt, das Lösungsmittel schonend bis zum Versiegen destilliert und der Rückstand in Aceton (ca. 75 l) gelöst. Es wird ein Lösungsmittelaustausch zu Aceton (6 Destillationsvorgänge mit je ca. 130 1) durchgeführt. Das Zielprodukt wird anschließend durch Zugeben in Wasser ausgefällt, durch Zentrifugieren isoliert und im Vakuumtrockner getrocknet. So werden insgesamt 16,5 kg *(S)-{8-Fluor-2-[4-(3-methoxyphenyl)piperazin-1-yl]-3-(2-methoxy-5-trifluormethylphenyl)-3,4-dihydrochinazolin-4-yl}essigsäure* als amorpher Feststoff erhalten, entsprechend 96,4 % der Theorie.
¹H NMR (300 MHz, d₆-DMSO): δ = 7,53 (d, ²*J* = 8,4, 1H), 7,41 (brs, 1H), 7,22 (d, ²*J* = 8,5, 1H), 7,09-7,01 (m, 2H), 6,86 (m, 2H), 6,45 (dd, ²*J* = 8,2, ³*J* = 1,8, 1H), 6,39-6,34 (m, 2H), 4,87 (t, ²*J* = 7,3, 1 H), 3,79 (brs, 3H), 3,68 (s, 3H), 3,50-3,38 (m, 4H), 2,96-2,75 (m, 5H), 2,45-2,40 (m, 1H) ppm;
MS (API-ES-neg.): m/z = 571 [(M-H), 100 %];
HPLC (Methode 1): R_{T} = 15,1 min;
HPLC (Methode 2): 99,8 % e.e.; Pd (ICP): <1 ppm.

### B.) Ausführungsbeispiele

### Kristallisationsversuche

Kristallisationsversuche zur Auffindung eines geeigneten kristallinen Salzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure wurden durchgeführt. Die Kristallisationsversuche erfolgten ausgehend von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure und der jeweiligen Säure entweder durch Aufschlämmen im jeweils angegebenen Lösungsmittel für eine Woche bei 20°C oder durch eine Kristallisation durch Abkühlen/Abdampfen ausgehend von einer Lösung, die für 4 Stunden bei 50°C gehalten wurde, gefolgt von einem langsamen Abkühlen auf 20°C mit einer Geschwindigkeit von 3°C/Stunde.

Die Ergebnisse für die Kristallisationsversuche sind nachstehend in Tabelle 1 angegeben wobei die Abkürzung API für (*S*)-{8-Fluor-2-[4-(3-methoxyphenyl)piperazin-1-yl]-3-[2-methoxy-5-(trifluormethyl)phenyl]-3,4-dihydrochinazolin-4-yl}essigsäure steht.

API" ist das Akronym für "active pharmaceutical ingredient" (aus dem Englischen für aktiver Arzneiwirkstoff).

**Tabelle 1**

| Kristallisationsversuche unter Verwendung saurer Gegenionen | | | | |
|---|---|---|---|---|
| **Gegenionen** | **Verhältnis API: Gegenionen** | **Verfahren** | **Lösungsmittel** | **Ergebnis (XRPD)** |
| HCL | 1:2 | Abkühlen | Aceton, Acetonitril, Methanol, THF | amorph |
| | | Aufschlämmen | Wasser, Acetonitril, Methanol und Ethanol | |
| Citronensäure | 1:1 | Abkühlen | Aceton, Acetonitril, Methanol, THF | amorph |
| | | Aufschlämmen | Wasser, Acetonitril Methanol und Ethanol | |
| Phosphorsäure | 1:1 | Abkühlen | Aceton, Acetonitril, Methanol, THF | amorph |
| | | Aufschlämmen | Wasser, Acetonitril, Methanol und Ethanol | |
| Gluconsäure | 1:1 | Abkühlen | Aceton, Acetonitril, Methanol, THF | amorph |
| | | Aufschlämmen | Wasser, Acetonitril Methanol und Ethanol | |
| Milchsäure | 1:1 | Abkühlen | Aceton, Acetonitril, Methanol, THF | amorph |
| | | Aufschlämmen | Wasser, Acetonitril Methanol und Ethanol | |
| Maleinsäure | 1:1 | Abkühlen | Aceton, Acetonitril, Methanol, THF | amorph |
| | | Aufschlämmen | Wasser, Acetonitril Methanol und Ethanol | |
| Bernsteinsäure | 1:1 | Abkühlen | Aceton, Acetonitril, Methanol, THF | amorph |
| | | Aufschlämmen | Wasser, Acetonitril Methanol und Ethanol | |
| Schwefelsäure | 1:1 | Abkühlen | Aceton, Acetonitril, Methanol, THF | amorph |
| | | Aufschlämmen | Wasser, Acetonitril Methanol und Ethanol | |
| Weinsäure | 1:1 | Abkühlen | Aceton, Acetonitril, Methanol, THF | amorph |
| | | Aufschlämmen | Wasser, Acetonitril Methanol und Ethanol | |
| Benzoesäure | 1:1 | Abkühlen | Aceton, Acetonitril, Methanol, THF | amorph |
| | | Aufschlämmen | Wasser, Acetonitril Methanol und Ethanol | |
| Fumarsäure | 1:1 | Abkühlen | Aceton, Acetonitril, Methanol, THF | amorph |
| | | Aufschlämmen | Wasser, Acetonitril Methanol und Ethanol | |
| Maleinsäure | 1:1 | Abkühlen | Aceton, Acetonitril, Methanol, THF | amorph |
| | | Aufschlämmen | Wasser, Acetonitril Methanol und Ethanol | |
| Methansulfonsäure | 1:1 | Abkühlen | Aceton, Acetonitril, Methanol, THF | amorph |
| | | Aufschlämmen | Wasser, Acetonitril Methanol und Ethanol | |

Was bei diesen Experimenten auffällig ist, ist dass es sich allgemein als äußerst schwierig erwiesen hat, kristalline Säuresalze von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure herzustellen, wobei die Kristallisation zumindest anfänglich vollkommen fehlgeschlagen ist. Im Lauf der weiteren Entwicklung wurde allerdings gefunden, dass kristalline Salze sowohl von Benzolsulfonsäure als auch Toluolsulfonsäure gewonnen werden konnten. Die gefundenen Besylat- und Tosylatsalze haben sich dabei als leicht und mit hoher Reinheit herstellbar erwiesen. Ferner hat sich aus den Röntgendiffraktogrammen gezeigt, dass diese Salze ohne Einlagerung von Lösungsmittelmolekülen kristallisieren.

### Ausführungsbeispiele

### Beispiel 1

### Besylatsalz von (S)-{8-Fluor-2-[4-(3-methoxyphenyl)piperazin-1-yl]-3-[2-methoxy-5-(trifluormethyl)phenyl]-3,4-dihydrochinazolin-4-yl}essigsäure

235,00 g (0,41 mol) *(S)*-[8-Fluor-2-[4-(3-methoxyphenyl)piperazin-1-yl]-3-[2-methoxy-5-(trifluormethyl)phenyl]-3,4-dihydrochinazolin-4-yl}essigsäure (Beispiel 6A) werden in 1645 ml Aceton gelöst und zu der erhaltenen Mischung werden 16,45 ml Wasser zugegeben. Die entstandene gelbliche Lösung wird filtriert und 64,92 g (0,41 mol) Benzolsulfonsäure als Feststoff in Portionen zugegeben. Die erhaltene Lösung wird auf etwa 40°C erwärmt und bei dieser Temperatur werden geeignete Impfkristalle zugegeben. Die resultierende Lösung wird unter Rühren auf Raumtemperatur abkühlen gelassen und die erhaltene Suspension wird auf 0-5°C gekühlt und bei dieser Temperatur für weitere 2 h gerührt. Der erhaltene Feststoff wird abfiltriert, 2x mit Aceton (100 ml, 0°C) gewaschen und bei 60°C zur Gewichtskonstanz getrocknet. Auf diese Weise werden insgesamt 243,87 g (81,4% der Theorie) der Zielverbindung erhalten.

Von dem im Beispiel 1 erhaltenen kristallinen Feststoff wurde auf einem Siemens Pulverdiffraktometer D5000 unter den folgenden Bedingungen ein Pulver-XRD-Diffraktogramm aufgenommen, das in Fig. 1 dargestellt ist.

Die Peaklisten für das Salz von Beispiel 1 sowie für das Salz von Beispiel 2 sind nachstehend in Tabelle 2 wiedergegeben.

### Messbedingungen

Kupferanode (Wellenlänge 1,5418 Ä)
Spannung: 4000 V, Strom: 30 mA
Sekundärer Graphitmonochromator,
variable (Theta-abhängige) Divergenz und Antidiffusionsabschirmung
Appertur des Detektors: 0,2 mm
6 x 10 mm effektive Probenoberfläche
Scanning 0,02° (2 Theta), 2 Sec.

### Beispiel 2

### Tosylatsalz von (S)-{8-Fluor-2-[4-(3-methoxyphenyl)piperazin-1-yl]-3-[2-methoxy-5-(trifluormethyl)phenyl]-3,4-dihydrochinazolin-4-yl}essigsäure

235,00 g (0,41 mol) *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure (Beispiel 6A) werden in 1645 ml Aceton gelöst und zu der Lösung werden 16,45 ml Wasser zugegeben. Die erhaltene gelbliche Lösung wird filtriert und 78,07 g (0,41 mol) feste p-Toluolsulfonsäure Monohydrat werden bei etwa 36°C portionsweise zugegeben. Die Lösung wird unter Rühren auf Raumtemperatur abkühlen gelassen und die erhaltene Suspension wird dann auf 0-5°C abgekühlt und bei dieser Temperatur für weitere 2 h gerührt. Der Feststoff wird abgetrennt, 2x mit Aceton (100 ml, 0°C) gewaschen und bei 60°C bis zur Gewichtskonstanz getrocknet. Auf diese Weise wurden insgesamt 248,11 g (79,3% der Theorie) des gewünschten Endprodukts erhalten.

Von dem im Beispiel 2 erhaltenen kristallinen Feststoff wurde unter den gleichen Bedingungen wie für das Beispiel 1 ein Pulver-XRD-Diffraktogramm aufgenommen, das in Fig. 2 dargestellt ist.

**Tabelle 2:**

| **2 Theta** | |
|---|---|
| **Beispiel 1** | **Beispiel 2** |
| 6,9 | 6,2 |
| 8,6 | 6,9 |
| 9,1 | 8,8 |
| 10,1 | 11,7 |
| 10,6 | 13,3 |
| 10,9 | 14,8 |
| 11,4 | 15,3 |
| 11,7 | 16,5 |
| 12,6 | 17,0 |
| 12,9 | 17,2 |
| 13,9 | 17,9 |
| 14,2 | 18,1 |
| 14,9 | 18,7 |
| 15,5 | 19,6 |
| 15,8 | 19,8 |
| 16,9 | 20,2 |
| 17,3 | 20,7 |
| 18,2 | 21,0 |
| 18,6 | 21,4 |
| 18,8 | 21,8 |
| 19,1 | 22,5 |
| 20,2 | 23,0 |
| 21,0 | 23,2 |
| 21,3 | 23,7 |
| 21,9 | 24,0 |
| 22,2 | 24,8 |
| 22,8 | 25,6 |
| 23,1 | 26,2 |
| 23,6 | 26,4 |
| 23,9 | 26,7 |
| 24,2 | 27,7 |
| 24,7 | 28,3 |
| 25,5 | 28,6 |
| 25,9 | 29,4 |
| 26,2 | 30,0 |
| 26,5 | 31,8 |
| 26,9 | 34,8 |
| 27,2 | 36,3 |
| 27,9 | 36,7 |
| 28,2 | 38,9 |
| 28,6 | 39,8 |
| 29,0 | |
| 29,4 | |
| 29,9 | |
| 30,6 | |
| 31,1 | |
| 31,5 | |
| 32,6 | |
| 33,1 | |
| 33,7 | |
| 34,4 | |
| 34,9 | |
| 36,6 | |
| 37,5 | |
| 38,1 | |
| 38,9 | |

### C.) Ausführungsbeispiele

### Reinheit

Die erfindungsgemäßen Verbindungen S(+)-8-Fluor-2-[4-{3-methoxyphenyl)piperazin-1-yl]-3-[2-methoxy-5-(trifluormethyl}phenyl]-3,4-dihydrochinazolin-4-yl}essigsäure Besylat und S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure Tosylat wurden mittels unten stehender HPLC (Methode 4) auf ihre chemische Reinheit hin untersucht.

In dem Kontext der vorliegenden Erfindung bezeichnet der Ausdruck "chemische Reinheit" den Grad des Stoffmengenanteils der oben genannten Salze im Vergleich zum gesamten Stoffgemisch. Die unerwünschten Stoffe werden als Verunreinigung(en) bezeichnet.

### Beispiel aa)

Synthese von S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure Besylat: 235 g (0,41 mol) des besagten Besylats wurden in 1645 ml Aceton und 16,45 ml Wasser gelöst. Die auf diese Weise erhaltene gelbliche Lösung wurde filtriert und mit 64,92 g (0,41 mol) festförmiger Benzolsulfonsäure portionsweise bei ca. 40°C behandelt. Unter Rühren wurde die erhaltene klare Lösung auf Raumtemperatur abgekühlt. Die daraufhin erhaltene Suspension wurde unter zweistündigem Rühren weiter auf 0°C - 5°C abgekühlt. Der feste Bestandteil davon wurde isoliert, zwei Mal mit Aceton gewaschen (je 100 ml, bei 0°C) und getrocknet bei 60°C, um so 243,87 g (0,334 mol, 81,4%) kristallines S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure Besylat zum Zweck der Reinheitsbestimmung zu erhalten.

### Beispiel bb)

Synthese von S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure Tosylat: 235 g (0,41 mol) des besagten Tosylats wurden in 1645 ml Aceton und 16,45 ml Wasser gelöst. Die auf diese Weise erhaltene gelbliche Lösung wurde filtriert und mit 78,07 g (0,41 mol) festförmigem p-Toluolsulfonsäure Monohydrat portionsweise bei ca. 36°C behandelt. Unter Rühren wurde die erhaltene Lösung auf Raumtemperatur abgekühlt. Die daraufhin erhaltenen Suspension wurde unter zweistündigem Rühren weiter auf 0°C - 5°C abgekühlt. Der feste Bestandteil davon wurde separiert, zwei Mal mit Aceton gewaschen (je 100 ml, bei 0°C) und getrocknet bei 60°C, um so 248,11 g (0,325 mol, 79,3%) kristallines S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure Tosylat zum Zweck der Reinheitsbestimmung zu erhalten.

### Reinheitsbestimmung

**Methode 4** (HPLC): Instrument: HP 1050 mit UV-Detektion; Säule: Phenomenex-Prodigy ODS (3) 100A, 150 mm x 3 mm, 3 µm; Eluent A: (1,36 g KH₂PO₄ + 0,7 ml H₃PO₄; 85%) / l Wasser; Eluent B: Acetonitril; Gradient: 0 min 20% B, 40 min 45% B, 50 min 80% B; 50 min 80% B, 65 min 80% B, 75 min 20% B; Fluss: 0,5 ml/min; Temp.: 55°C; Injektionsvolumen 3 µl, UV-Detektion: 210 nm / BW: 4 nm; Temperatur Auto Sampler 5°C.

Je 22 mg der jeweiligen Testsubstanz der erhaltenen Salze nach Beispiel aa); Beispiel bb) wurden in 50 ml Acetonitril gelöst (c = ca. 0,44 mg/ml). Als entsprechende Referenzlösungen wurden je 22 mg S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure in 50 ml Acetonitril gelöst (c = ca. 0,44 mg/ml).

Als Referenzlösungen der bekannten Verunreinigungen wurden je 5 mg von Di-p-Toluoyl-D-Weinsäure, S-Chinazolylpiperazin, Chinazolin Ethylester und Chinazolyldipiperazin separat mit Acetonitril auf 50 ml vermengt. Je 1 ml der Referenzlösungen für bekannte Verunreinigungen wurde daraufhin separat in 10 ml Acetonitril gelöst (c = ca. 0,01 mg/ml).

Ferner wurden je 10 ml der Referenzlösung S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure mit je 1 ml jeder der Referenzlösungen für die oben benannten Verunreinigungen gemischt.

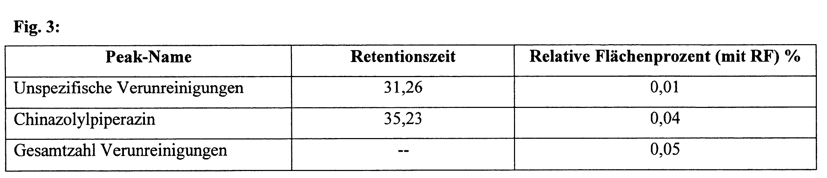

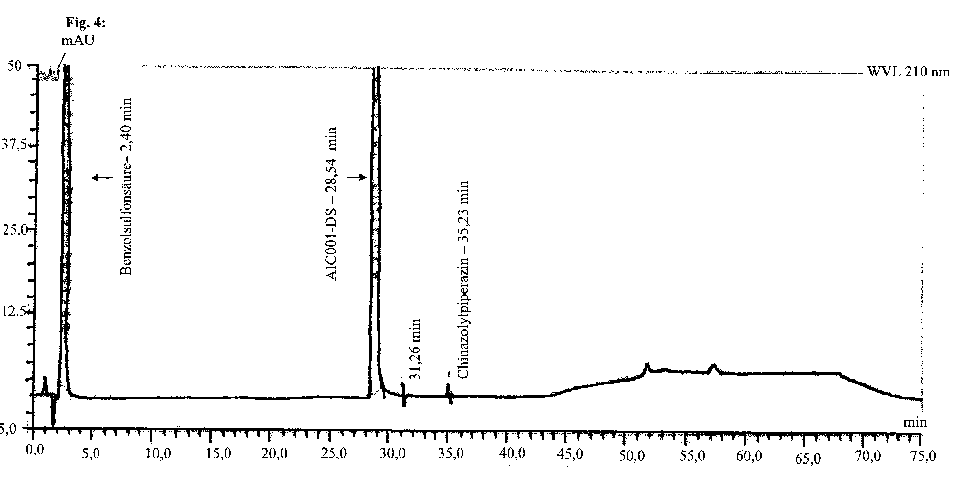

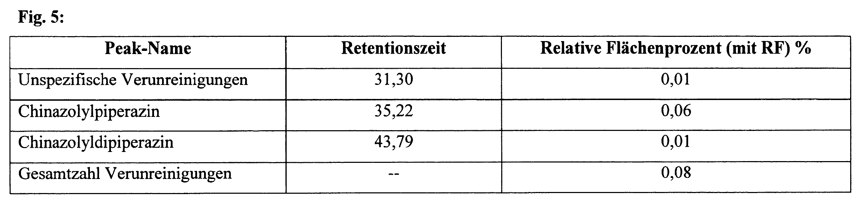

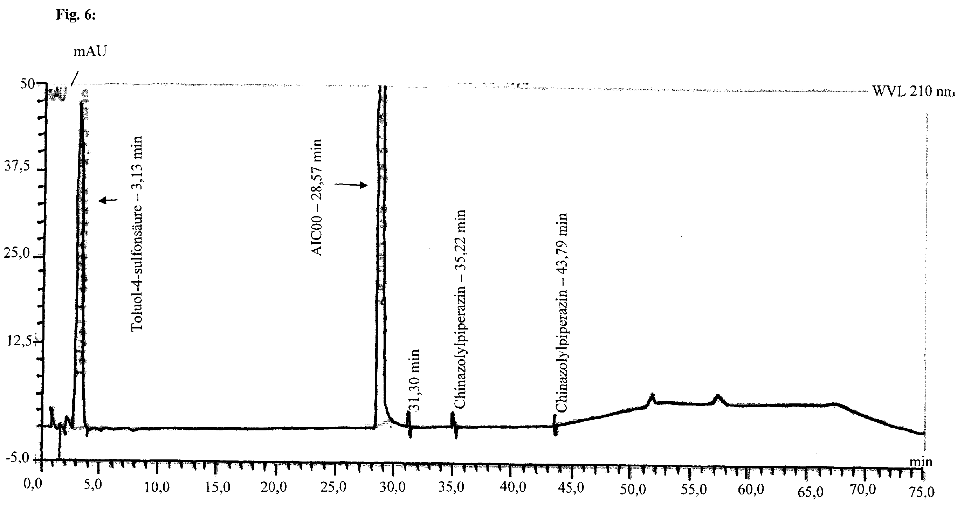

Die Evaluierung der HPLC-Analyse wurde anhand der sogenannten relativen Flächenprozent-Methode, welche die entsprechenden Response-Faktoren (RF) mit in Betracht zieht, durchgeführt (RF = 1,02 für Chinalzolylpiperazin und 0,81 für Chinazolyldipiperazin). Ergebnisse siehe in Fig. 3 und Fig. 4 für S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure Besylat und siehe Fig. 5 und Fig. 6 für S(+)-8-Fluor-2-[4-(3-methoxyphenyl)piperazin-1-yl]-3-[2-methoxy-5-(trifluormethyl)phenyl]-3,4-dihydrochinazolin-4-yl}essigsäure Tosylat.

### D.) Pharmazeutische Zubereitung

### Tablette:

### Zusammensetzung:

128 mg des Salzes von Beispiel 1 (entspricht 100 mg Wirkstoff), 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min, gemischt. Diese Mischung wird mit einer üblichen Tablettenpresse verpresst (Format der Tablette siehe oben). Als Richtwert für die Verpressung wird eine Presskraft von 15 kN verwendet.

## Patentansprüche

1. Salz von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Solvat davon, ausgewählt aus der Gruppe bestehend aus den kristallinen Besylatsalzen von {8-Fluor-2-[4-(3-methoxyphenyl)piperazin-1-yl]-3-[2-methoxy-5-(trifluormethyl)phenyl]-3,4-dihydrochinazolin-4-yl}essigsäure, den kristallinen Tosylatsalzen von {8-Fluor-2-[4-(3-methoxyphenyl)piperazin-1-yl]-3-[2-methoxy-5-(trifluormethyl}phenyl]-3,4-dihydrochinazolin-4-yl}essigsäure und deren Solvaten.

2. Salz nach Anspruch 1, wobei es sich um das Monobesylatsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Solvat davon handelt.

3. Salz nach Anspruch 2, wobei es sich um das Monobesylatsalz von {8-Fluor-2-[4-(3-methoxyphenyl)piperazin-1-yl]-3-[2-methoxy-5-(trifluormethyl}phenyl]-3,4-di-hydrochinazolin-4-yl}essigsäure handelt.

4. Salz nach Anspruch 3, **dadurch gekennzeichnet, dass** es im Pulver-XRD-Diffraktogramm charakteristische Peaks bei etwa 6,9, 10,1 und 22,2 Grad 2Theta zeigt.

5. Salz nach Anspruch 1, wobei es sich um das Monotosylatsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Solvat davon handelt.

6. Salz nach Anspruch 5, wobei es sich um das Monotosylatsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure handelt.

7. Salz nach Anspruch 6, **dadurch gekennzeichnet, dass** es im Pulver-XRD-Diffraktogramm charakteristische Peaks bei etwa 6,9 und 20,7 Grad 2Theta zeigt.

8. Verfahren zur Herstellung eines Salzes nach einem der Ansprüche 1 bis 7 mit den folgenden Schritten:
a.) Lösen von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder eines Solvats davon in einer Mischung aus Wasser und zumindest einem (C₃-C₆)-Alkanon, ggf. unter Erwärmen.
b.) Zugabe von Benzolsulfonsäure oder Toluolsulfonsäure zu der in Schritt a.) erhaltenen Lösung,
c.) Abkühlen der in Schritt b.) erhaltenen Lösung um die Kristallisation des Salzes bzw. eines Solvat eines Salzes auszulösen,
d.) Abtrennen des in Schritt c.) auskristallisierten Salzes oder Solvats davon, und e.) Trocknen des in Schritt d.) erhaltenen Salzes oder Solvats.

9. Verfahren zur Aufreinigung von {8-Fluor-2-[4-(3-methoxyphenyl)piperazin-1-yl]-3-[2-methoxy-5-{trifluormethyl}phenyl]-3,4-dihydrochinazolin-4-yl}essigsäure mit den folgenden Schritten:
1.) Umsetzen von {8-Fluor-2-[4-(3-methoxyphenyl)piperazin-1-yl]-3-[2-methoxy-5-{trifluormethyl)phenyl]-3,4-dihydrochinazolin-4-yl}essigsäure mit Benzolsulfonsäure bzw. Toluolsulfonsäure in einem Lösungsmittel um ein kristallines Salz gemäß Anspruch 1 zu erhalten,
2.) Isolieren des in Schritt 1.) erhaltenen Salzes,
3.) Behandeln des in Schritt 2.) isolierten Salzes mit einer Pufferlösung bei pH 5 bis 7 zum Freisetzen der zwitterionischen Form von {8-Fluor-2-[4-(3-methoxyphenyl)piperazin-1-yl]-3-[2-methoxy-5-(trifluormethyl)phenyl]-3,4-dihydrochinazolin-4-yl}essigsäure, und
4.) Isolieren der in Schritt 3.) erhaltenen zwitterionischen Form von {8-fluor-2-[4-(3-methoxyphenyl)piperazin-1-yl]-3-[2-methoxy-5-(trifluormethyl)phenyl]-3,4-dihydrochinazolin-4-yl}essigsäure.

10. Salz nach einem der Ansprüche 1 bis 7 zur Verwendung in einem Verfahren zur Behandlung und/oder Prophylaxe von Krankheiten, insbesondere von Virusinfektionen, vorzugsweise von Infektionen mit HCMV oder einem anderen Vertreter der Gruppe der Herpes viridae.

11. Arzneimittel enthaltend ein Salz nach einem der Ansprüche 1 bis 7 in Kombination mit zumindest einem pharmazeutisch annehmbaren Hilfsstoff.

12. Arzneimittel nach Anspruch 11 zur Verwendung in einem Verfahren zur Behandlung und/oder Prophylaxe von Virusinfektionen, vorzugsweise von Infektionen mit HCMV oder einem anderen Vertreter der Gruppe der Herpes viridae

13. Verwendung eines Salzes nach einem der Ansprüche 1 bis 7 zur Herstellung eines Medikaments zur Behandlung und/oder Prophylaxe von Virusinfektionen vorzugsweise von Infektionen mit HCMV oder einem anderen Vertreter der Gruppe der Herpes viridae.

14. Salz nach einem der Ansprüche 1 bis 7 zur Verwendung in einem Verfahren zur Bekämpfung von Virusinfektionen, vorzugsweise von Infektionen mit HCMV oder einem anderen Vertreter der Gruppe der Herpes viridae, in Menschen und Tieren, das die Verabreichung des besagten Salzes oder eines Arzneimittels nach Anspruch 11 oder 12 an einen Menschen oder ein Tier, der (das) eine solche Behandlung benötigt, aufweist.

## Claims

1. A salt of {8-fluoro-2-[4-(3-methoxyphenyl)piperazine-1-yl]-3-[2-methoxy-5-(trifluoromethyl) phenyl]-3,4-dihydroquinazoline-4-yl}acetic acid or a solvate thereof, selected from the group consisting of the crystalline besylate salts of {8-fluoro-2-[4-(3-methoxyphenyl)piperazine-1-yl]-3-[2-methoxy-5-(trifluoromethyl)phenyl]-3,4-dihydroquinazoline-4-yl}acetic acid, the crystalline tosylate salts of {8-fluoro-2-[4-(3-methoxyphenyl)piperazine-1-yl]-3-[2-methoxy-5-(trifluoromethyl)phenyl]-3,4-dihydroquinazoline-4-yl}acetic acid and solvates thereof.

2. The salt according to Claim 1, which is the monobesylate salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid or a solvate thereof.

3. The salt according to Claim 2, which is the monobesylate salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid.

4. The salt according to Claim 3, **characterized in that** it shows characteristic peaks at about 6.9, 10.1 and 22.2 degrees 2theta in the X-ray powder diffractogram (XRD).

5. The salt according to Claim 1, which is the monotosylate salt of {8-fluoro-2-[4-{3-methoxyphenyl)piperazine-1-yl]-3-[2-methoxy-5-(trifluoromethyl)phenyl]-3,4-dihydroquinazoline-4-yl}acetic acid or a solvate thereof.

6. The salt according to Claim 5, which is the monotosylate salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid.

7. The salt according to Claim 6, **characterized in that** it shows characteristic peaks at about 6.9 and 20.7 degrees 2theta in the X-ray powder diffractogram (XRD).

8. A method for producing a sait according to any one of Claims 1 to 7 using the following steps:
a.) Dissolving {8-fluoro-2-[4-(3-methoxyphenyl)piperazine-1-yl]-3-[2-methoxy-5-(trifluoromethyl)phenyl]-3,4-dihydroquinazoline-4-yl}acetic acid or a solvate thereof in a mixture of water and at least one (C₃-C₆) alkanone, if necessary under heat.
b.) Adding benzenesulfonic acid or toluenesulfonic acid to the solution obtained in step a.),
c.) Cooling down the solution obtained in step b.) in order to initiate the crystallization of the salt or of a solvate of a salt,
d.) Separating the crystallized-out salt or solvate thereof obtained in step c.), and
e.) Drying the salt or solvate obtained in step d.).

9. A method for purifying 18-fluoro-2-[4-(3-methoxyphenyl)piperazine-1-yl]-3-[2-methoxy-5-(trifluoromethyl)phenyl]-3,4-dihydroquinazoline-4-yl}acetic acid using the following steps:
1.) Reacting {8-fluoro-2-[4-(3-methoxyphenyl)piperazine-1-yl]-3-[2-methoxy-5-(trifluoromethyl)phenyl]-3,4-dihydroquinazoline-4-yl}acetic acid in a solvent with benzenesulfonic acid or toluenesulfonic acid to obtain a crystalline salt according to Claim 1,
2.) Isolating the salt obtained in step 1.),
3.) Treating the isolated salt obtained in step 2.) with a buffer solution at pH 5 to 7 to release a zwitterionic form of {8-fluoro-2-[4-(3-methoxyphenyl)piperazine-1-yl]-3-[2-methoxy-5-(trifluoromethyl]phenyl]-3,4-dihydroquinazoline-4-yl}acetic acid, and
4.) Isolating the zwitterionic form of {8-fluoro-2-[4-(3-methoxyphenyl)piperazine-1-yl]-3-[2-methoxy-5-(trifluoromethyl)phenyl]-3,4-dihydroquinazoline-4-yl}acetic acid obtained in step 3.).

10. The salt according to any one of Claims 1 to 7 for use in a method of treatment and/or prophylaxis of diseases, in particular of virus infections, preferably human cytomegalovirus (HCMV) infections or infections with another representative of the *herpes viridae* group.

11. A drug containing a salt according to any one of Claims 1 to 7 in combination with at least one pharmaceutically acceptable excipient.

12. The drug according to Claim 11 for use in a method of treatment and/or prophylaxis of virus infections, preferably human cytomegalovirus (HCMV) infections or infections with another representative of the *herpes viridae* group.

13. The use of a salt according to any one of Claims 1 to 7 for the manufacture of a medicament for the treatment and/or prophylaxis of virus infections, preferably human cytomegalovirus (HCMV) infections or infections with another member of the *herpes viridae* group.

14. The salt according to any one of Claims 1 to 7 for use in a method for combating virus infections, preferably human cytomegalovirus (HCMV) infections or infections with another representative of the *herpes viridae* group, in humans and animals by administering said salt or a drug according to Claims 11 or 12 to a human or an animal who or which requires such a treatment.

## Revendications

1. Sel de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl} ou un solvate de celui-ci choisi parmi le groupe comprenant les sels cristallins de bésylate de l'acide acétique de {8-fluoro-2-[4-(3-méthoxyphényle)pipérazine-1-yl]-3-[2-méthoxy-5-(trifluorométhyle)phényle-3,4-dihydroquinazoline-4-yl}, les sels cristallins de tosylate de l'acide acétique de {8-fluoro-2-[4-(3-méthoxyphényle)pipérazine-1-yl]-3-[2-méthoxy-5-(trifluorométhyl)phényle]-3,4-dihydroquinazoline-4-yl} et les solvates de ceux-ci.

2. Sel selon la revendication 1, qui concerne le sel de monobésylate de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl} ou un solvate de celui-ci.

3. Sel selon la revendication 2, qui concerne le sel de monobésylate de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl}.

4. Sel selon la revendication 3, **caractérisé en ce qu'**il présente des pics caractéristiques d'environ 6,9, 10,1 et 22,2 degrés 2thêta dans le diffractogramme de poudre par rayons X (XRD).

5. Sel selon la revendication 1, qui concerne le sel de monotosylate de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl} ou un solvate de celui-ci.

6. Sel selon la revendication 5, qui concerne le sel de monotosylate de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl}.

7. Sel selon la revendication 6, **caractérisé en ce qu'**il présente des pics caractéristiques d'environ 6,9 et 20,7 degrés 2thêta dans le diffractogramme de poudre par rayons X (XRD).

8. Procédé de production d'un sel selon l'une quelconque des revendications 1 à 7, consistant à :
a.) Dissoudre l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl} ou un solvate de celui-ci dans un mélange d'eau et d'au moins un alcanone (C3-C6), si nécessaire, sous apport de chaleur.
b.) Ajouter de l'acide benzènesulfonique ou de l'acide toluènesulfonique à la solution obtenue à l'étape a.),
c.) Refroidir du solvant de la solution obtenue à l'étape b.) pour lancer la cristallisation du sel ou d'un solvate d'un sel,
d.) Séparer le sel cristallisé ou le solvate de celui-ci obtenu à l'étape c.) ; et
e.) Sécher le sel ou le solvate obtenu à l'étape d.).

9. Procédé de purification de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl}, consistant à :
1.) Faire réagir l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl} dans un solvant avec l'acide benzènesulfonique ou l'acide toluènesulfonique pour obtenir un sel cristallin selon la revendication 1 ;
2.) Isoler le sel obtenu à l'étape 1.) ;
3.) Traiter le sel obtenu à l'étape 2) avec une solution tampon ayant un pH de 5 à 7 pour libérer la forme zwitterionique de l'acide acétique de {8-fluoro-2-[4-{3-méthoxyphényle)pipérazine-1-yl]-3-[2-méthoxy-5-(trifluorométhyle)phényle]-3,4-dihydroquinazoline-4-yl} ; et
4.) Isoler la forme zwitterionique de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl} obtenue à l'étape 3.

10. Sel selon l'une des revendications 1 à 7, destiné à être utilisé dans une méthode de traitement et/ou de prévention de maladies, en particulier les infections virales, de préférence les infections au cytomégalovirus humain (HCMV) ou les infections par un autre membre de la famille des Herpes viridae.

11. Médicament contenant un sel selon l'une des revendications 1 à 7 en association avec au moins un excipient pharmaceutiquement acceptable.

12. Médicament selon la revendication 11, destiné à être utilisé dans une méthode de traitement et/ou de prévention d'infections virales, de préférence les infections au cytomégalovirus humain (HCMV) ou les infections par un autre membre de la famille des Herpes viridae.

13. Utilisation d'un sel selon l'une des revendications 1 à 7, pour la fabrication d'un médicament destiné au traitement et/ou à la prévention d'infections virales, de préférence les infections au cytomégalovirus humain (HCMV) ou les infections par un autre membre de la famille des Herpes viridae.

14. Sel selon les revendications 1 à 7, destiné à être utilisé dans une méthode de lutter contre les infections virales, de préférence les infections au cytomégalovirus humain (HCMV) ou les infections par un autre membre de la famille des Herpes viridae chez un être humain ou un animal, en administrant ledit sel ou un médicament selon les revendications 11 ou 12 à un humain ou à un animal qui a besoin d'un tel traitement.
